# EUROPEAN PATENT APPLICATION

(11) **EP 2 233 077 A1**
(43) Date of publication of application: **29.09.2010**
(21) Application number: 08857801.8
(22) Date of filing: 04.12.2008
(51) Int. Cl.: A61B 5/16, A61B 10/00, G06Q 50/00

(54) **PERSONALITY TESTING APPARATUS**

(30) Priority: 07.12.2007 JP 2007316703
(71) Applicant: Zaidanhojin Shin-Iryozaidan, Osaka 545-0053 (JP)
(72) Inventor: MAKI, Shuichi, Tondabayashi-shi Osaka 584-0069 (JP); HATA, Tadayo, Osaka-shi Osaka 545-0053 (JP)
(74) Representative: Vossius & Partner
(86) International application number: PCT/JP2008/072040
(87) International publication number: WO 2009/072554

(57) **Abstract**

A personality testing apparatus which tests the personality of a subject has a display means (1) which displays question items (10) of MMPI (Minnesota Multiphasic Personality Inventory) to the subject, an input means (2) for inputting answers of the subject to the question items (10), a storage means (31) which stores test data obtained from the answers of the subject, a data processing means (3) which reads the test data, calculates the test data according to each criterion and generates a result table (20) obtained by aggregating the calculation result. The apparatus enables the subject to easily answer to MMPI, and makes it possible to accurately and speedily score and aggregate the test data.

## Description

### TECHNICAL FIELD

The present invention relates to a personality testing apparatus for testing personality.

### BACKGROUND ART

Conventionally, MMPI (Minnesota Multiphasic Personality Inventory) for testing personality is a test in which a subject is made to answer question items in an answer sheet to test the personality based on the answers to the question items.
This test requires, when the answers are aggregated and scored, a scoring apparatus including a wooden scoreboard on which the answer sheet filled with the answers of the subject is placed and a color translucent resin sheet (score panel) overlapping the top face of the answer sheet on the scoreboard. The scoring apparatus makes it possible to score (calculate) each scale from the answers by placing the answer sheet filled with the answers of the subject on the scoreboard, overlapping the sheet (score panel) on the top face of the answer sheet, and counting the marks and the like on the answer sheet from colorless transparent windows formed in the score panel to enable. Furthermore, the scored results are filled in a predetermined recording sheet manually (by a grader).

While, Patent Document 1 discloses the system in which, in the Kraepelin test for testing personality, a subject is made to answer questions on an answer sheet such as a mark sheet with writing instruments and an aggregator let the answers be read with a reader or the like.
Patent Document 1: Japanese Patent No. 3385268.

### DISCLOSURE OF THE INVENTION

### TECHNICAL PROBLEMS TO BE SOLVED

However, the MMPI (Minnesota Multiphasic Personality Inventory) for testing personality involves many manual operations such as aggregating, and scoring of the answers (test data) and inputting of the data, which raises the possibility of errors in the aggregation, scoring and input of the data and also makes it less time efficient. In particular, in the answer (mark) reading operation, a different score panel (sheet) is required to be used depending on the sex of the subject or a scale in order to count the marks. Thus, the scoring operation is troublesome, and counting errors such as miscounts might occur.
In addition, a subject might miss answers or answer incorrectly.

Therefore, an object of the present invention is to provide a personality testing apparatus which is easier for a subject to answer MMPI (Minnesota Multiphasic Personality Inventory) and allows faster and more reliable scoring and, aggregation of the test data.

### MEANS TO SOLVE THE PROBLEMS

The personality testing apparatus according to the present invention used to achieve the above object is a personality testing apparatus for testing personality of a subject. The apparatus includes display means for displaying a question item of MMPI (Minnesota Multiphasic Personality Inventory) to the subject, input means for inputting an answer of the subject to the question item, storage means for storing test data obtained from the answer of the subject, and data processing means for reading out the test data, calculating the test data according to a scale, and preparing a result table obtained by aggregating the calculation result.

Furthermore, a personality testing apparatus for testing personality of a subject include display means for displaying a question item of MMPI (Minnesota Multiphasic Personality Inventory) to the subject, input means for inputting an answer of the subject to the question item, storage means for storing test data obtained from the answers o the subject, data processing means for reading out the test data, calculating the test data according to a scale, and preparing a result table obtained by aggregating the calculation result, and output means capable of outputting the result table as a printed material.

In addition, in the personality testing apparatus, the data processing means calculates a validity scale and a clinical scale from the test data obtained from the answers of the subject to the question item, and prepares the result table obtained by aggregating the calculation result.

Furthermore, the question item corresponds to the calculation of a plurality of the scales.
Moreover, the personality testing apparatus further includes voice output means for conveying the question item to the subject through voice.

### EFFECT OF THE INVENTION

With the personality testing apparatus of the invention, scoring can be performed without using conventional scoring apparatus such as the scoreboard and the score panel. The apparatus can reduce the possibility of mistaken answers by the subject and can eliminate the possibility of scoring errors caused by miscounts or recording errors by a grader. Furthermore, the scoring operation can be accurately and speedily performed. Namely, accurate and reliable MMPI (Minnesota Multiphasic Personality Inventory) can be performed.

### BRIEF DESCRIPTION OF DRAWINGS

Fig. 1 is a schematic configuration diagram showing a first embodiment.
Fig. 2 is a flowchart showing a first embodiment.
Fig. 3 shows an example of a display screen of display means.
Fig. 4 shows an example of a display screen of the display means.
Fig. 5 shows an example of a display screen of the display means.
Fig. 6 shows an example of a display screen of the display means.
Fig. 7 shows an example of a display screen of the display means.
Fig. 8 shows an example of a display screen of the display means.
Fig. 9 shows an example of a display screen of the display means.
Fig. 10 shows an example of a display screen of the display means.
Fig. 11 shows an example of a display screen of the display means.
Fig. 12 shows an example of a display screen of the display means.
Fig. 13 shows an example of a display screen of the display means.
Fig. 14 shows an example of a display screen of the display means.
Fig. 15 shows an example of a display screen of the display means.
Fig. 16 shows an example of a display screen of the display means.
Fig. 17 shows an example of a first result table.
Fig. 18 shows an example of a second result table.
Fig. 19 is a flowchart showing a first embodiment.
Fig. 20 shows an example of a display screen of the display means.
Fig. 21 shows an example of a display screen of the display means.
Fig. 22 shows an example of a display screen of the display means.
Fig. 23 is a schematic configuration diagram showing a second embodiment.
Fig. 24 is a schematic configuration diagram showing a third embodiment.

### BRIEF DESCRIPTION OF REFERENCE NUMERALS

- 1: Display means
- 2: Input means
- 3: Data processing means
- 4: Output means
- 5: Voice output means
- 10: Question item
- 20: Result table
- 30: Printed material
- 31: Storage means

### BEST MODE FOR CARRYING OUT THE INVENTION

Hereinafter, the present invention will be described in detail with reference to drawings showing embodiments thereof.

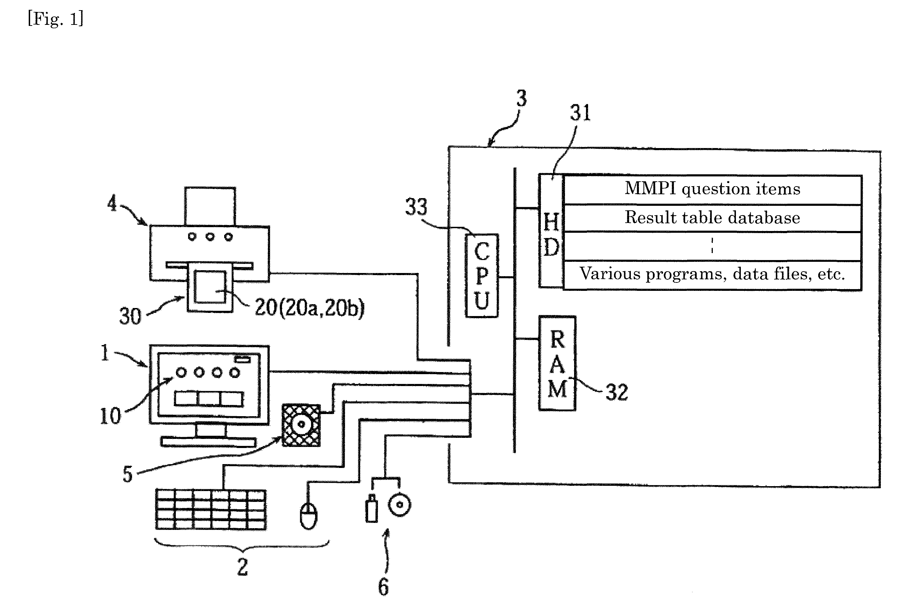

Fig. 1 is a schematic configuration diagram showing a first embodiment of the personality testing apparatus of the invention. Data processing means 3 includes storage means 31 such as a hard disk (HD) that stores 550 question items 10 of MMPI (Minnesota Multiphasic Personality Inventory), 383 question items of short form MMPI, various programs, and the like. The data processing means 3 includes a temporary storage means, 32 such as a RAM (random access memory) or ROM (read-only memory), that temporarily stores data such as a calculation result or the program, and calculation control means 33, such as a CPU (central processing unit), that can control the input and output of the data, read and write the data on the storage means 31, and calculate the data. The temporary storage means 32 is capable of transmitting the stored content through the calculation control means 33 to the storage means 31 to each other.

Furthermore, the data processing means 3 is connected through a wired or wireless network to display means 1 that includes a display screen such as a display monitor capable of displaying the MMPI question item 10 read from the storage means 31, input means 2 that includes a keyboard, mouse, and the like capable of inputting the answer to the question item 10 displayed on the display means 1 and data such as a name, output means 4 such as a printer capable of outputting, as a printed material 30, a result table 20 and the like such as test data (answer data of a subject to the question item 10) and a scored result read from the storage means 31, and voice output means 5 such as a speaker capable of outputting the MMPI question items 10 and the like through voice so that the data, input and output signals, and the like could be sent or received.
Furthermore, the data processing means 3 can write the data on an external storage medium 6 such as a CD, DVD, or USB memory.

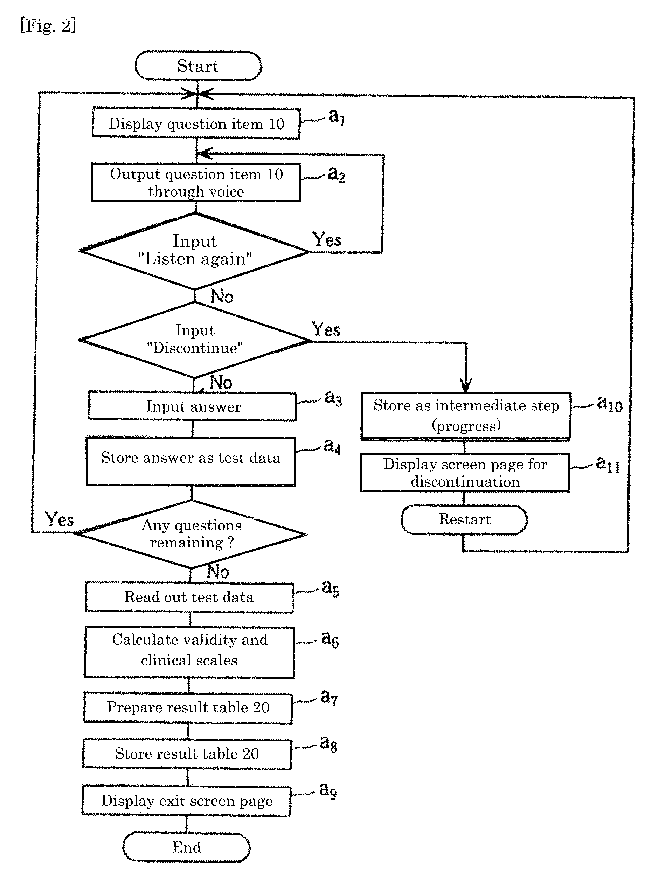

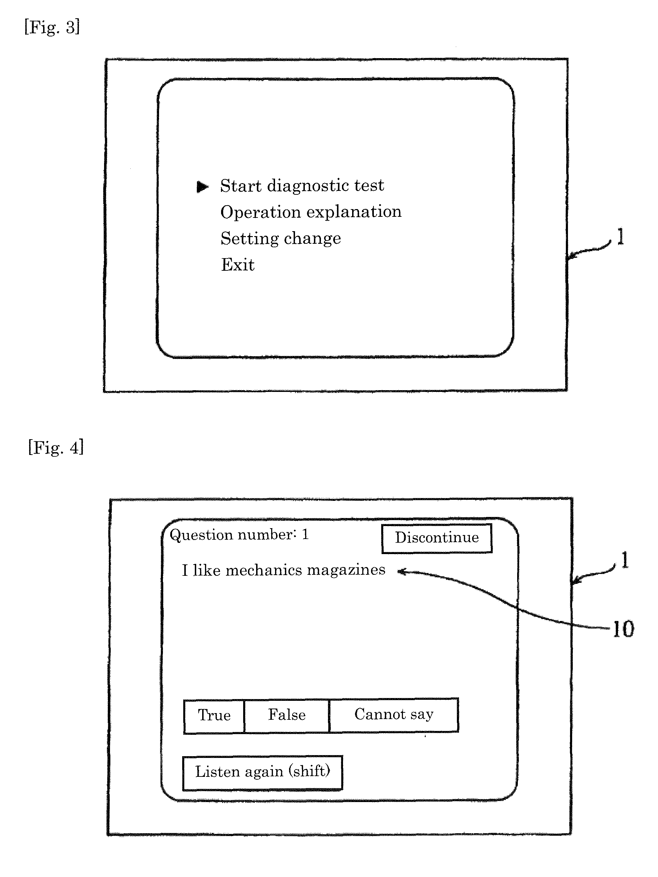

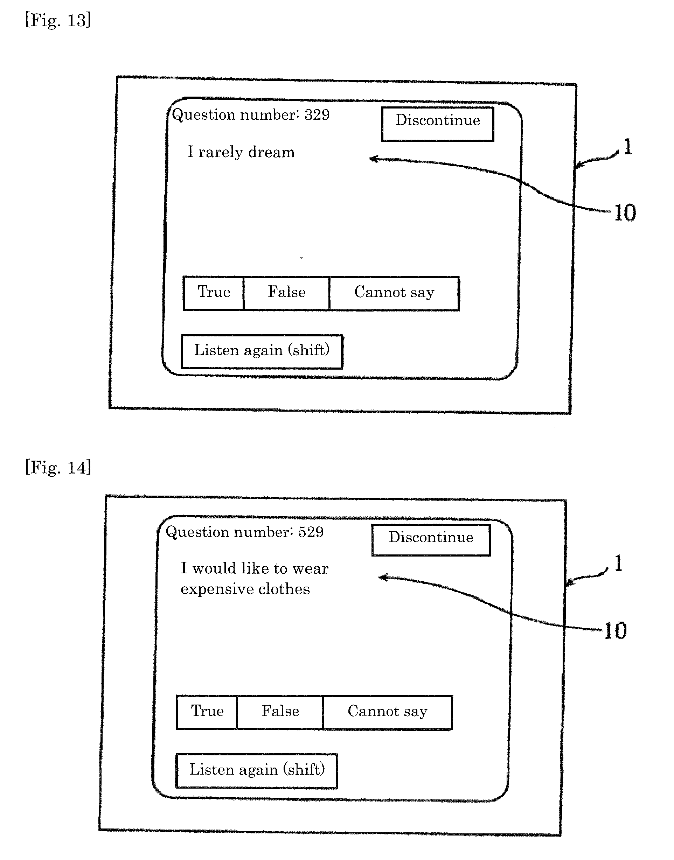

Specific procedures of the test, calculation, and aggregation will be described with reference to a flowchart shown in Fig. 2 and each example of the display screen of the display means 1 shown in Fig. 3 to Fig. 16.
In advance, the data processing means 3 certifies a subject by a password and login name or personal identification data (ID) such as a name or sex of the subject, and then displays a screen page of the question asking which test to start, the MMPI test (MP test) or the short form MMPI test, on the display means 1 (not shown in the drawings).
When the "MMPI test" is selected and input through the input means 2, a start screen of the MMPI test is displayed on the display means 1.
The display means 1 displays items such as "Start diagnostic test" on the display screen as shown in Fig. 3. The data processing means 3 starts the test and aggregation following the selection and input of "Start diagnostic test" through the input means 2 (by a mouse click or a predetermined key operation on the keyboard).

In Step a₁ in Fig. 2, the calculation control means 33 of the data processing means 3 displays the question item 10: "I like mechanics magazines" as the first question item 10 stored in the storage means 31 on the display means 1 as shown in Fig. 4 as well as display the answers to the question item 10: "True", "False", and "Cannot say".
In addition, the display screen of the display means 1 displays optional items: "Discontinue" for selecting to discontinue the test and "Listen again (Shift)" for selecting to output the displayed question item 10 from the voice output means 5 through voice.

In next Step a₂, the data processing means 3 makes the voice output means 5 output the question item 10: "I like mechanics magazines" through voice to convey the question item 10 to the subject.

In Step a₃, one of the answers of "True", "False", and "Cannot say" is input through the input means 2 to the data processing means 3.
In Step a₄, the calculation control means 33 of the data processing means 3 makes the storage means 31 store the answer as the test data (namely, the data processing means 3 stores the test data). At the time, the data processing means 3 stores the test data associating with information such as the subject ID and login name or the subject name and sex.

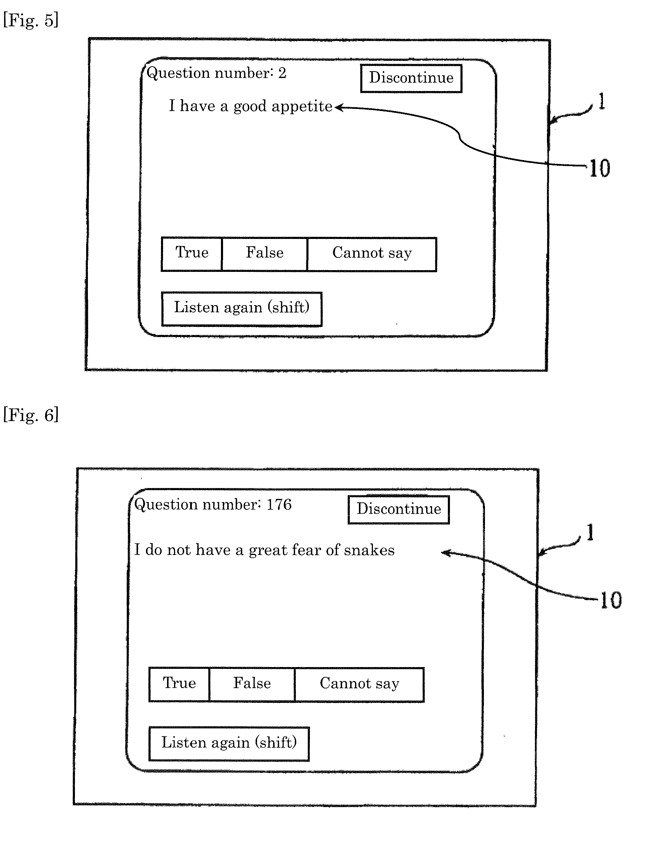

The data processing means 3 goes back to Step a₁ when any question items 10 remain, and displays the next question item 10: "I have a good appetite" on the display screen of the display means 1 as shown in Fig. 5.
Furthermore, in Step a₂, the voice output means 5 outputs the question item 10 through voice to convey it to the subject.

Here, when "Listen again (Shift)" is input by a mouse click or by pressing the shift key of the keyboard of the input means 2 to the data processing means 2, the data processing means 3 goes back to Step a₂ and makes the voice output means 5 output the question item 10: "I have a good appetite" through voice again.

When one of the answers of "True", "False", and "Cannot say" is input through the input means 2, the calculation control means 33 of the data processing means 3 stores the answer as the test data in the storage means 31 (Step a₄).

Since the question items 10 are not completely answered, the data processing means 3 sequentially displays each of the MMPI question items 10 such as "I do not have a great fear of snakes" and "I would like to do hunting" on the display screen of the display means 1 as shown in Fig. 6 to Fig. 7, and the storage means 31 sequentially stores each answer as the test data.

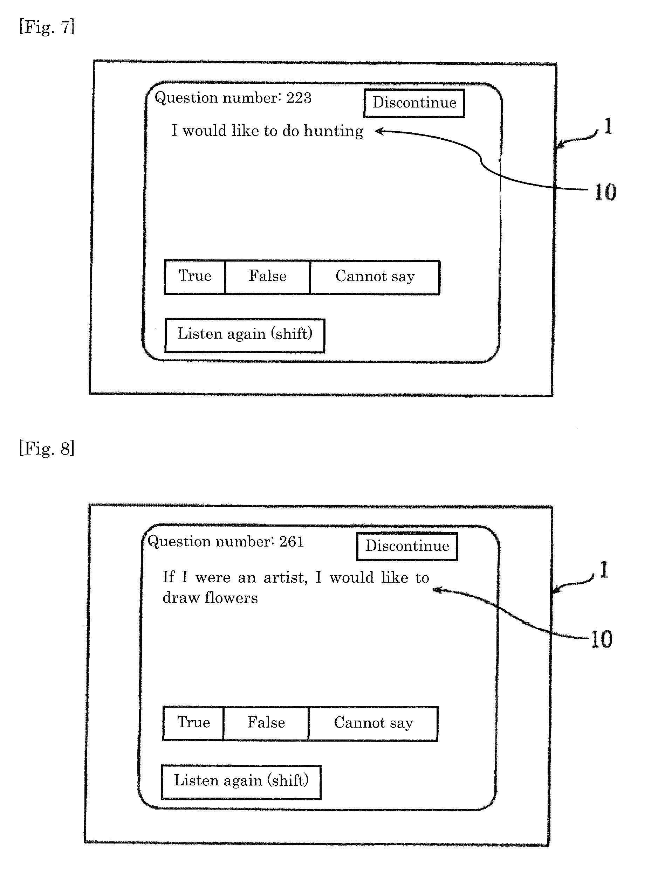

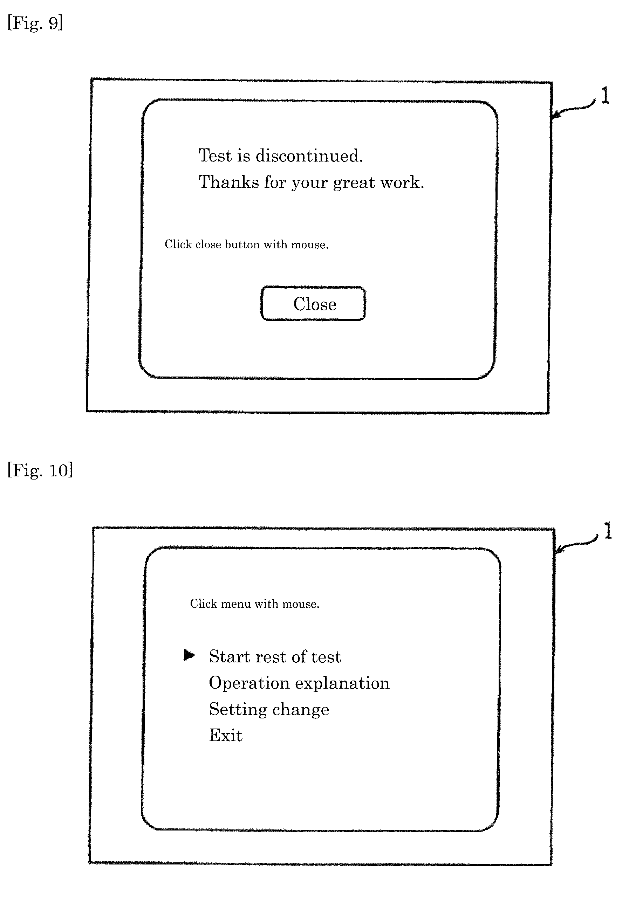

Here, during the procedure (from Step a₁ to Step a₄) in which the display of the question item 10, the storage of the answer as the test data, and the display of the next question item 10 are repeated, for example, at the time of the question: "If I were an artist, I would like to draw flowers" shown in Fig. 8, if the subject becomes too tired to answer the question and inputs (selects) "Discontinue" through the input means 2 to the data processing means 3, the calculation control means 33 of the data processing means 3 stores the progress of the test and the like (for example, how many question items 10 have been answered) in the storage means 31 (Step a₁₀). In next Step a₁₁, the data processing means 3 displays "Close" as a screen page for discontinuation on the display means 1 as shown in Fig. 9. By inputting (selecting) "Close" through the input means 2, the test is discontinued.

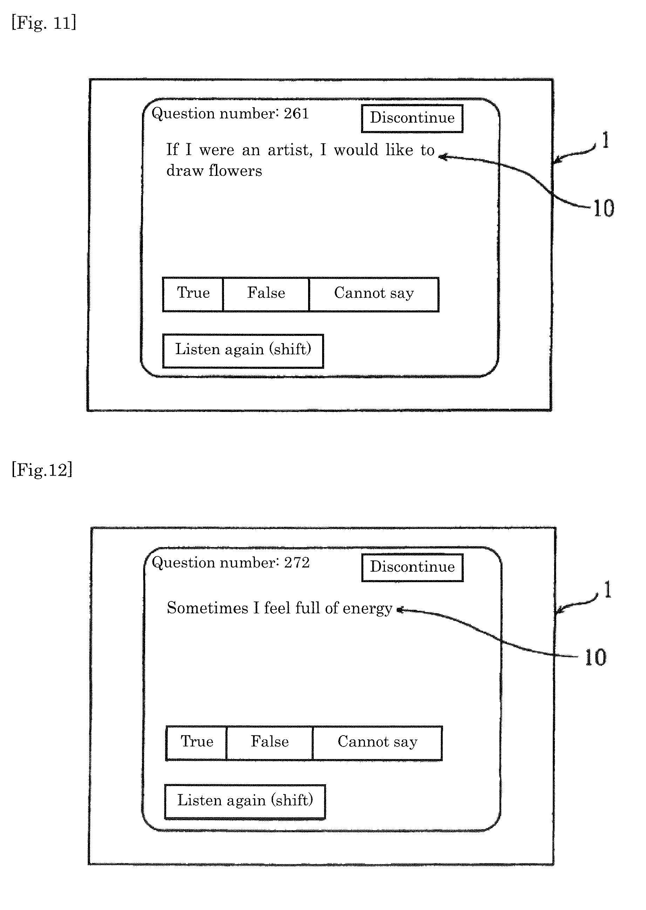

Then, as shown in Fig. 10, the data processing means 3 that has certified the subject by the password and login name or the personal identification data (ID) such as the name or sex of the subject displays the display screen for restart on the display means 1. When "Start rest of test" is input (selected) through the input means 2, the calculation control means 33 of the data processing means 3 reads out the unanswered question item 10: "If I were an artist, I would like to draw flowers" from the storage means 31, goes back to Step a₁, and displays "If I were an artist, I would like to draw flowers" on the display means 1 again as shown in Fig. 11. When the answer is input through the input means 2 (the subject answers one of "True", "False", and "Cannot say" with the input means 2), the calculation control means 33 of the data processing means 3 stores the answer as the test data in the storage means 31 (Step a₄).

Since the question items 10 are not completely answered, the data processing means 3 goes back to Step a₁ again, and Step a₁ to Step a₄ are sequentially repeated. That is, as shown in Fig. 12 to Fig. 14, the data processing means 3 sequentially displays each of the MMPI question items 10 such as "Sometimes I feel full of energy", "I rarely dream", and "I would like to wear expensive clothes" on the display screen of the display means 1.

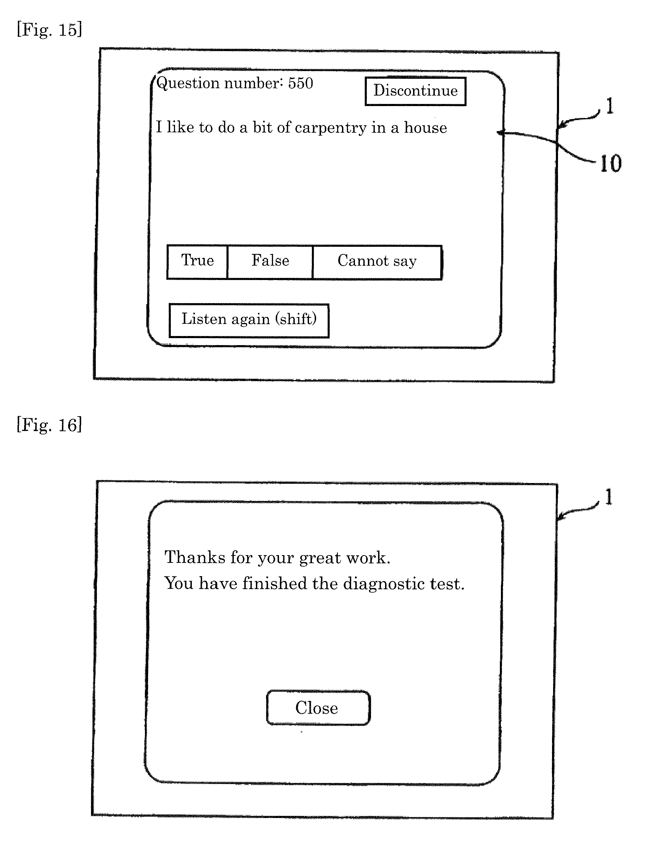

Then, when the answer of the last question item 10 of the 550 items: "I like to do a bit of carpentry in the house" as shown in Fig. 15 is input, in Step a₅, the calculation control means 33 of the data processing means 3 reads out all of the test data obtained from the answers of the subject from the storage means 31. Next, in Step a₆, the test data is aggregated to calculate the validity scales and the clinical scales of the test data. Then, in Step a₇, the data processing means 3 prepares a result table 20 from the calculation result. In Step a₈, the result table 20 is stored as a data file in the storage means 31. In Step a₉, the display means 1 displays "Thanks for your great work. You have finished the diagnostic test" and "Close" as shown in Fig. 16.
"Close" is input through the input means 2, and the data processing means 3 terminates the test, calculation, and aggregation.

Here, the calculation of the validity scales and the clinical scales of the test data in Step a₆ will be described.
The validity scales detect bias in the test taker's attitude and include a total of four scales:
the ? scale that is the number of items answered "Cannot say" and detects the validity of the interpretation;
the L scale that is the number of predetermined answers to predetermined 15 items and detects comparatively simple bias in the test taker's attitude when the subject seeks to present themselves in a favorable light;
the F scale that is the number of predetermined answers to predetermined 64 items, and becomes higher score when a subject overacts, for example, malingering, and is the index showing the bias in the test-taker's attitude and psychiatric degree; and
the K scale that is the number of predetermined answers to predetermined 30 items, and represents a defensive, self-critical attitude, and the like, and is also used for a modified score to a part of the clinical scales.
The predetermined answer is either answer of "True" or "False" that should be added to the scales (scored as the scales).

Each clinical scale is named after a psychopathological type that is used when the degree of each scale's characteristics is extremely high, and includes a total of ten scales:
the hypochondriasis (Hs) scale that is the number of predetermined answers to predetermined 33 items and shows the tendency of having excess fear of one's own physical condition;
the depression (D) scale that is the number of predetermined answers to predetermined 60 items and shows the tendency of depressed mood and suppression of thinking and action;
the hysteria (Hy) scale that is the number of predetermined answers to predetermined 60 items and shows the tendency of readily developing particularly physical conversion symptoms;
the psychopathic deviate (Pd) scale that is the number of predetermined answers to predetermined 50 items and shows the tendency of readily developing antisocial acts among psychopathic personalities;
the sexual (Mf) scale that is the number of predetermined answers to predetermined 60 items and shows a masculinity/femininity tendency with interest;
the paranoia (Pa) scale that is the number of predetermined answers to predetermined 40 items and shows the tendency of readily having delusion such as suspicion;
the psychasthenia (Pt) scale that is the number of predetermined answers to predetermined 48 items and shows the tendency of obsessive-compulsive neurosis;
the schizophrenia (Sc) scale that is the number of predetermined answers to predetermined 78 items and shows the tendency of odd activity, hallucination and delusion, and abnormal thinking;
the hypomania (Ma) scale that is the number of predetermined answers to predetermined 46 items and shows the tendency of excessive thinking and activity; and
the social introversion (Si) scale that is the number of predetermined answers to predetermined 70 items and shows the tendency of disliking social contact.

That is, in Step a₆ shown in Fig. 2, the data processing means 3 scores predetermined answers of the question items 10 corresponding to the calculation standard of each of the L, F, and K scales among the 550 question items (the number of answers of "True" or "False" to be added is counted). Furthermore, as the ? scale, the number of answers of "Cannot say" is counted. In addition, the number of answers to be added in the predetermined question items 10 corresponding to the calculation standard of each clinical scale is counted. Each counted number is stored in the storage means 31 as the raw score of each scale.

Then, the data processing means 3 adds the value (additional value) that is obtained by multiplying the K scale by each predetermined ratio to each of the Hs, Pd, Pt, Sc, and Ma scales to calculate each modified raw score of the Hs, Pd, Pt, Sc, and Ma scales in order to improve the certainty of the test (to increase the discrimination power).
The modified raw score of the Hs scale is calculated by adding the raw score of the Hs scale to 0.5-fold raw score of the K scale. That is, it is represented by Hs + 0.5K (in the result table 20, Hs + .5K).
The modified raw score of the Pd scale is calculated by adding the raw score of the Pd scale to 0.4-fold raw score of the K scale. That is, it is represented by Pd + 0.4K (in the result table 20, Pd + .4K).
The modified raw score of the Pt scale is calculated by adding the raw score of the Pt scale to 1.0-fold raw score of the K scale. That is, it is represented by Pt + 1.0K (in the result table 20, Pt + 1K).
The modified raw score of the Sc scale is calculated by adding the raw score of the Sc scale to 1.0-fold raw score of the K scale. That is, it is represented by Sc + 1.0K (in the result table 20, Sc + 1K).
The modified raw score of the Ma scale is calculated by adding the raw score of the Ma scale to 0.2-fold raw score of the K scale. That is, it is represented by Ma + 0.2K (in the result table 20, Ma + .2K).

Furthermore, each of the raw scores or modified scores is calculated by the calculation control means 33 with a predetermined conversion ratio (conversion equation) corresponding to each scale to calculate the score. In the first embodiment, the subject is a male. Specifically, the raw score of the depression (D) scale is 33 points, but it is converted to give a score of 75 points when the subject is male. Furthermore, the modified raw score (Sc + 1K) of the schizophrenia (Sc) scale is 36 points, and the modified raw score of 36 points is converted to give a score of 67 points when the subject is male. That is, in the case of the scale for which the modified raw score is calculated, not the raw score but the modified raw score is converted to calculate the score. Here, the conversion ratio (conversion equation) for converting the raw score or modified raw score to the score is different depending on whether the subject is a male or female. For example, for the K scale, a raw score of 0 to 30 points is converted to 19 to 80 points in males and to 17 to 81 points in females.

Namely, in Step a₆, the calculation of the validity and clinical scales is that the test data is aggregated and scored to calculate the raw score or modified raw score of each scale to calculate each score of the validity scale and clinical scale. Furthermore, in other words, aggregating and scoring the test data for calculating the raw score or modified raw score for each scale and then calculating each score of the validity scale and clinical scale is referred to as calculating the test data for each scale.

Here, some of the question items 10 correspond to the calculation (standard) of a plurality of the scales. For example, the question item 10: "Sometimes I feel full of energy" as shown in Fig. 12 is the question item 10 for detecting the F scale and the K scale and, when a subject answers "False", it is added to each of the F scale and the K scale as the raw score. Furthermore, as for the Mf scale, different answers are added depending on the sex (male or female). In the first embodiment, the subject is a male.

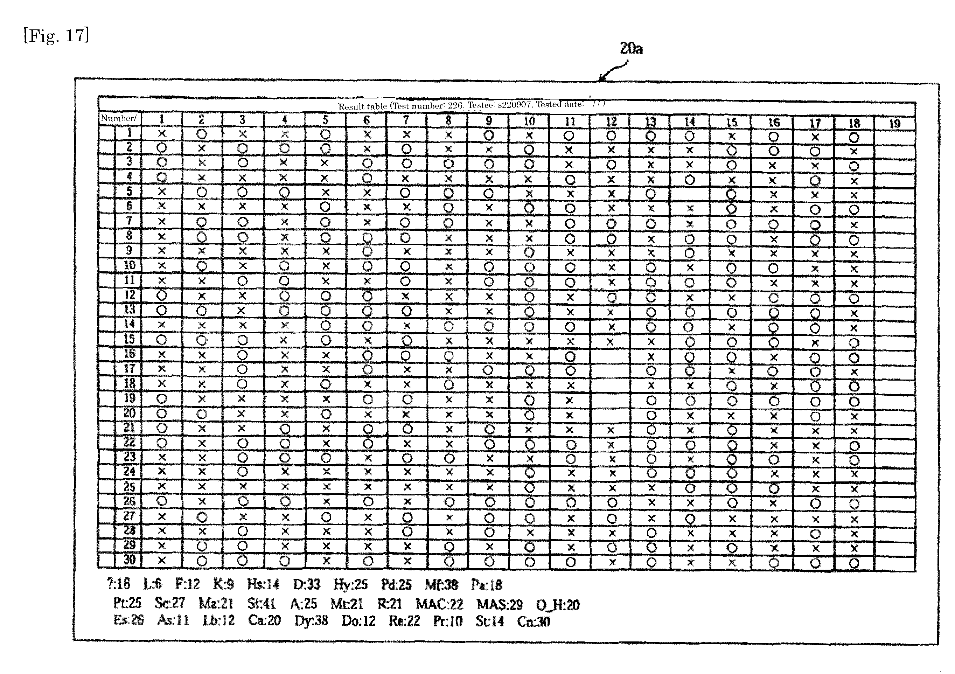

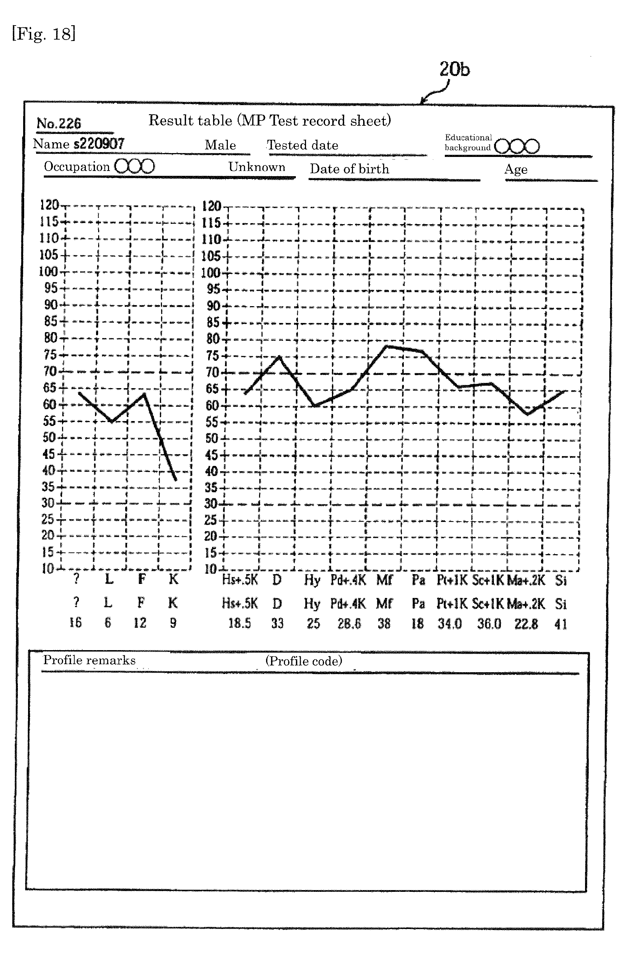

Next, the result tables 20 prepared in Step a₇ are a first result table 20a and a second result table 20b: as shown in Fig. 17, the first result table 20a is a list showing the answers to the question items 10 (test data) showing "True" as O, "False" as X, and "Cannot say" as a blank and the raw score of each scale; and as shown in Fig. 18, the second result table 20b is a line graph having a horizontal axis as the scale and a vertical axis as the score converted from the raw score or the modified raw score.
Namely, in Step a₇, the data processing means 3 aggregates the raw score, modified raw score, and score calculated from the test data for each scale to prepare the first and second result tables 20a and 20b (the result tables 20). When the line graph of the second result table 20b is prepared, the score of each scale that is converted from the raw score or modified raw score of each scale is plotted on the vertical axis. Furthermore, a line is not drawn between the validity scale and the clinical scale (that is, between the K scale and the Hs scale) in the line graph.

In Step a₈, the result tables 20 (the first and second result tables 20a and 20b) are stored as the data file in the storage means 31. In other words, the storage means 31 of the data processing means 3 stores the result tables 20 as the data file. Furthermore, the result tables 20 are associated with the subject ID, subject (testee) name, and the information such as sex (married or unmarried, educational background, date of birth, occupation, and age) to make a searchable database for storing (the result table database is prepared and stored).

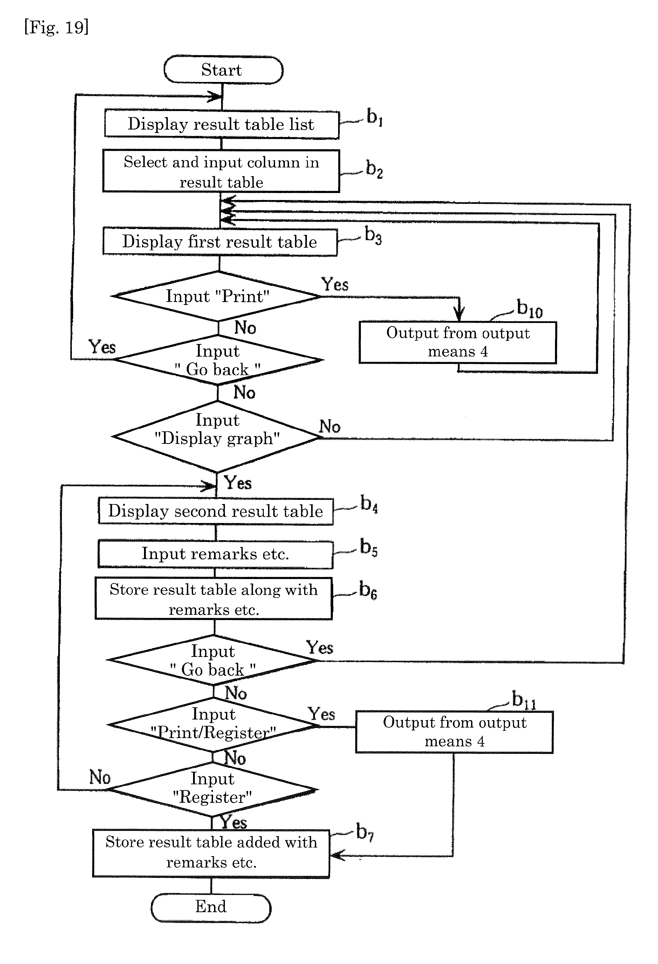

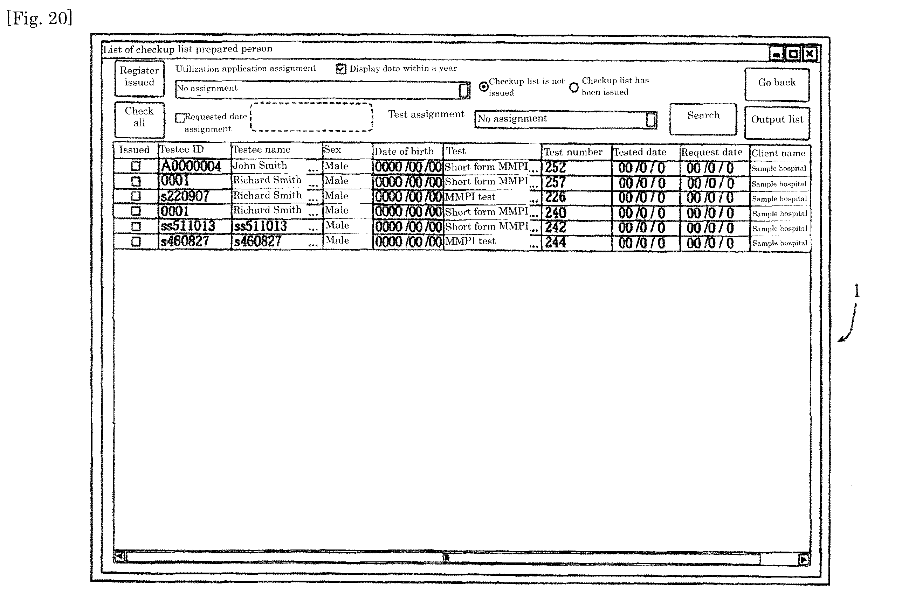

Next, with reference to a flowchart shown in Fig. 19 and examples of the display screen of the display means 1 shown in Fig. 20 to Fig. 22, a procedure for outputting the result table 20 and a procedure for registering and storing a remark in the result table 20 will be specifically described.

When the data processing means 3 certifies a tester (doctor or nurse) by the administrator's ID and password for tester and the like (different from the subject's ID and password), (in Step b₁ in Fig. 19) the data processing means 3 displays a list of the result table 20 (result table database) that is stored in association with the subject (testee) ID, the subject name, and the information such as sex on the display means 1 (as shown in Fig. 20).

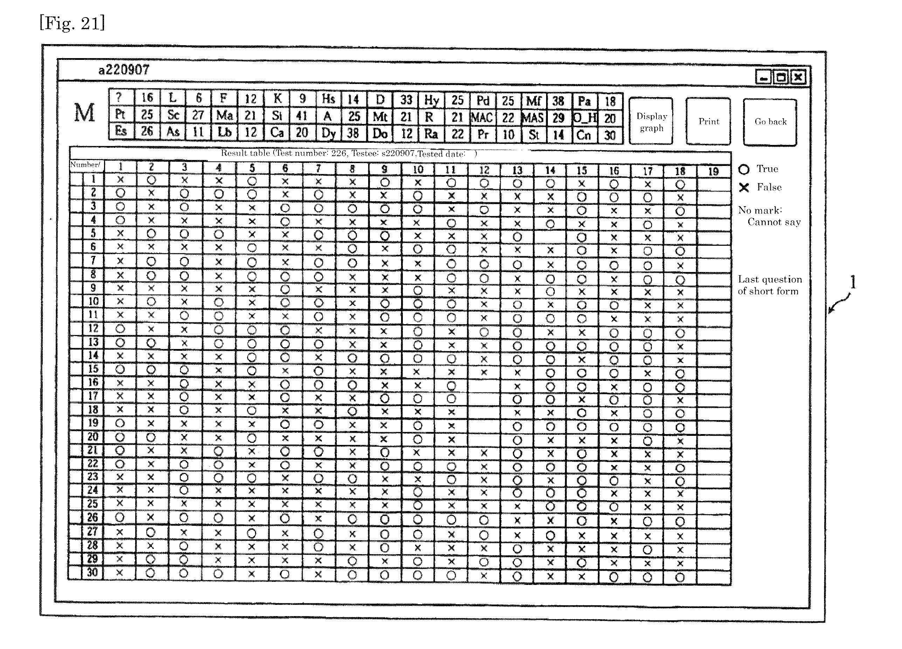

In Step b₂, as shown in Fig. 20, from the list displayed on the display screen of the display means 1, a column of the result table 20 of the subject (testee) to be displayed is selected and input through the input means 2 into the data processing means 3.
In Step b₃, the calculation control means 33 of the data processing means 3 reads out the data file of the first result table 20a from the storage means 31. The first result table 20a is displayed on the display screen of the display means 1 as shown in Fig. 21. In addition, the data processing means 3 displays "Go back (to Step b₁)", "Print", and "Display graph (the second result table 20b)" on the display means 1.

When "Print" is selected (input) from the input means 2, the data processing means 3 outputs the first result table 20a from the output means 4 as a printed material 30 as shown in Fig. 17 (Step b₁₀).

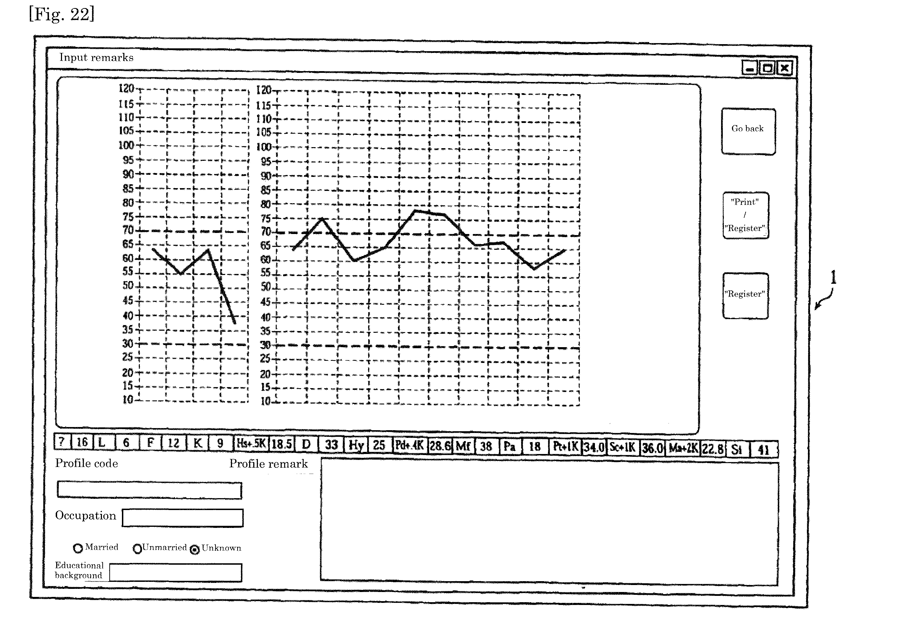

When "Display graph (the second result table 20b)" is input (selected) from the input means 2, in Step b₄, the data processing means 3 reads out the data file of the second result table 20b from the storage means 31 to display the second result table 20b on the display means 1 as shown in Fig. 22. Furthermore, the data processing means 3 displays "Go back (to Step b₃)", "Print/Register", and "Register" as the input selection items on the display means 1.

In Step b₅, a profile code, profile remarks, and the like are added in the result table 20 through the input means 2.
In Step b₆, the first and second result tables 20a and 20b (result tables 20) are stored along with the profile remarks and the like in the storage means 31.

Furthermore, when "Print/Register" is selected and input through the input means 2, the data processing means 3 outputs the second result table 20b as the printed material 30 from the output means 4 (Step b₁₁). In addition, when a comment has been input, the input content of the comment is stored along with the result table 20b in the storage means 31.

Furthermore, when "Register" is selected and input through the input means 2, the result tables 20 (first and second result tables 20a and 20b) that are added with the remarks to the result table database stored in the storage means 31 is stored (Step b₇).

Moreover, the stored test data and result tables 20 (first and second result tables 20a and 20b) can be read out from the storage means 31 and stored in an external storage medium 6.

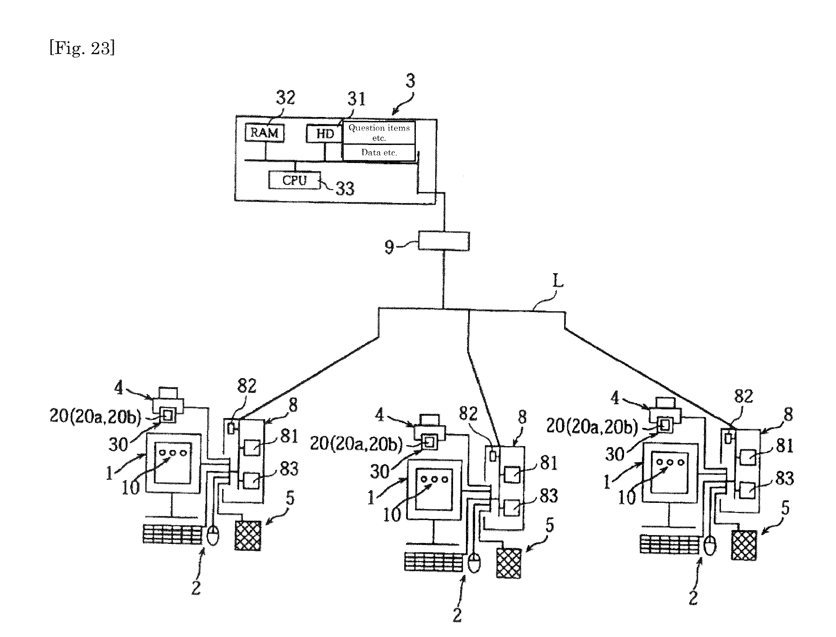

Next, Fig. 23 shows the schematic configuration diagram of a second embodiment.
In the second embodiment, the data processing means 3 connects through a wired or wireless network to a network L such as in-office LAN or in-hospital LAN through a repeater 9 such as a hub. In addition, the data processing means 3 is connected as a server through a wired or wireless network to a plurality of terminal information processing means 8 through the network L so that a plurality of different data could be sent and received.

Each information processing means 8 is connected to, in a similar manner as the data processing means 3 in the first embodiment, terminal display means 1 displaying the MMPI question items 10 (or the short form MMPI question items) to a subject, terminal input means 2 inputting the answers of the subject to the question items 10, terminal output means 4 capable of outputting the result table 20 as the printed material 30, and voice output means 5 conveying the question items 10 through voice.
Furthermore, the information processing means 8 includes a storage means 81, such as a hard disk (HD), capable of storing the data that is sent to or received from the data processing means 3. Moreover, the information processing means 8 includes information calculation control means 83, such as a CPU, capable of transmitting a command signal from the data processing means 3 to the terminal display means 1 and the terminal voice output means 5 for performance. Furthermore, the information calculation control means 83 is capable of transmitting input data from the terminal input means 2 to the data processing means 3. In addition, the information processing means 8 includes a temporary storage means 82, such as a RAM, capable of temporarily storing input data, sent and received data, and a receive command signal. The temporary storage means 82 is capable of transmitting the recorded content through the information calculation control means 83 to the storage means 81 to each other.

The data processing means 3 reads out the question item 10 data of MMPI (Minnesota Multiphasic Personality Inventory) from the storage means 3, sends the data to each terminal information processing means 8 whose ID and password are certified by the data processing means 3, and displays the question items 10 on each terminal display means 1 through the information processing means 8. Furthermore, the data processing means 3 makes each terminal voice output means 5 output the question items 10 through voice.

Furthermore, the data processing means 3 is capable of receiving the answer input through each terminal input means 2 in association with the ID. The data processing means 3 stores the received terminal test data in the storage means 31, calculates the scale in a similar manner as in the first embodiment (calculates the raw score, modified raw score, and score), prepares the result table 20 and stores it in the storage means 31. It then stores the result table 20 along with the remark and the like.

Furthermore, the data processing means 3 reads out the data file of the result tables 20 (first and second result tables 20a and 20b) from the storage means 31, sends the data file of the result tables 20 (first and second result tables 20a and 20b) added with the remarks and the like to the terminal information processing means 8 whose ID and password are certified by the data processing means 3, and displays the result table 20 on the terminal display means 1. Namely, it enables a subject in a distant place to take the test as well as to receive the result promptly. Moreover, the data processing means 3 is capable of independently sending the question items 10 according to the progress of each subject. In addition, it is capable of independently discontinuing the test with respect to each information processing means 8.

That is, the calculation control means 33 of the data processing means 3 sends the data such as the MMPI question items 10 and the selection items read out from the storage means 31 through the repeater 9 and the network L to the terminal information processing means 8, and displays the question items 10 on the terminal display means 1 through the information processing means 8. Furthermore, it makes the terminal voice output means 5 output the question items 10 through voice.

Next, the data such as the answers and question items 10 input through the terminal input means 2 is sent to the data processing means 3 through the information processing means 8. The data processing means 3 stores the input data as test data and sends the next question item 10. Furthermore, the data processing means 3 sends the voice output command, and when the answers (test data) for all question items 10 are obtained, the data processing means 3 prepares the result tables 20 (first and second result tables 20a and 20b) and stores it in a similar manner as in the first embodiment.
In a similar manner as in the first embodiment, the data processing means 3 sends commands such as the command to display the screen page for discontinuation and the command to the voice output means 5 to read the question item 10 again.

Furthermore, the data processing means 3 certifies the terminal information processing means 8 by the ID and password, sends the result tables 20 (first and second result tables 20a and 20b) added with the remarks and the like to the certified terminal information processing means 8, and displays the result tables 20 on the terminal display means 1. The output means 4 outputs the result tables 20 as the printed materials 30. Namely, the result is conveyed to the subject away from the data processing means 3.

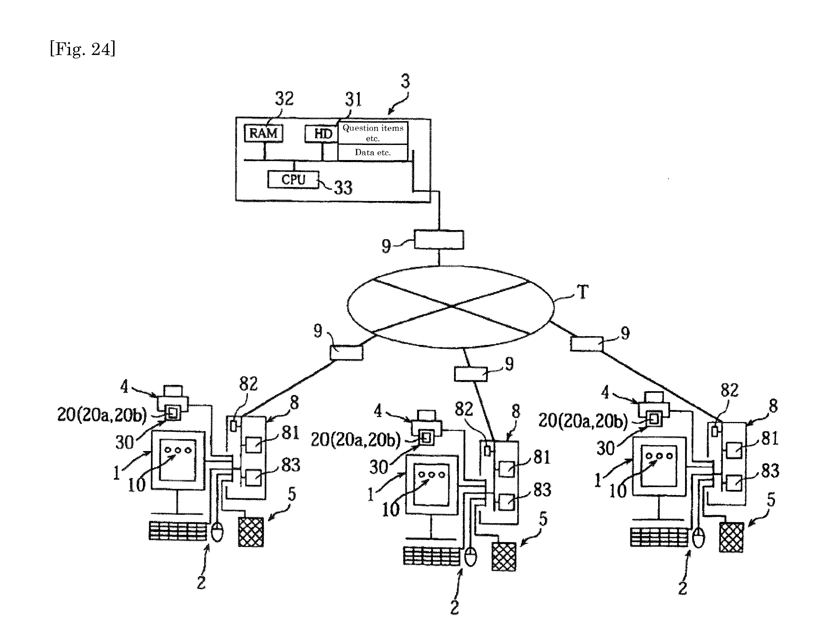

Next, Fig. 24 shows the schematic configuration diagram of a third embodiment.
In the third embodiment, the data processing means 3 connects through a wired or wireless network to a communication network T such as the Internet through a repeater 9 such as a modem. Furthermore, the data processing means 3 is connected, as a server, through a wired or wireless network to a plurality of terminal information processing means 8 through the communication network T and the repeater 9 so that a plurality of different data can be sent and received.

That is, in the third embodiment, the communication network T such as the Internet is used instead of the network L described in the second embodiment. Namely, an user who has obtained permission by the data processing means 3 as the server can take the MMPI test (MP test) or short form MMPI test using information processing means 8, display means 1, input means 2, output means 4, and voice output means 5 that are similar to those in the second embodiment and that are located in a place other than a hospital. Furthermore, even from a hospital (a plurality of other hospitals) without the data processing means 3, the subject can take the test if the subject's ID and password are given, and the administrator can diagnose (input the remark and the like and output the result table 20) if the administrator's ID and password are given. In other words, when the data processing means 3 is used as a server and the license system is introduced, the apparatus can be used in the world.

Design changes may be made to the present invention. For example, a remark data table storing a remark comment may be stored in the storage means 31. According to the change, when the score is out of a predetermined standard region (a score between 30 and 70 points), the calculation control means 33 of the data processing means 3 can read out the remark comment from the remark data table in the storage means 31 and display or output it along with the second result table 20b.
Furthermore, the procedure for outputting the first and second result tables 20a and 20b and the procedure for inputting the remarks may have a different sequence from the flow chart shown in Fig. 19. For example, the second result table 20b may be displayed on ahead. Moreover, the sequence of the procedures such as "Go back", "Print", and "Register" and Step b₅ where the remark is input may be interchanged.
Furthermore, the input means 2 may be a touch panel and the like unified with the display means 1.
Furthermore, the question items 10 may be freely increased or decreased. In addition, an original question item may be freely added.
Moreover, it is desirable that the raw scores and scores of additional scales are calculated to be indicated in the result table 20. The additional scales are the anxiety (A) scale, repression (R) scale, manifest anxiety (MAS) scale, ego strength (Es) scale, low-back pain (Lb) scale, parietal lobe/frontal lobe damage (Ca) scale, dependency (Dy) scale, dominance (Do) scale, social responsibility (Re) scale, prejudice (Pr) scale, social status (St) scale, control (Cn) scale, college maladjustment scale (Mt), MacAndrew alcoholism (MAC) scale, over-controlled-hostility (O-H) scale, alexithymia (As) scale, and the like.

As described above, the present invention is a personality testing apparatus for testing personality of a subject. Since the apparatus includes the display means 1 for displaying a MMPI (Minnesota Multiphasic Personality Inventory) question item 10 to the subject, the input means 2 for inputting an answer of the subject to the question item 10, the storage means 31 for storing test data obtained from the answers by the subject, and the data processing means 3 for reading out the test data, calculating the test data according to a scale, and preparing a result table 20 obtained by aggregating the calculation result, aggregation and scoring can be speedily performed without using the conventional scoring apparatus such as the scoreboard and the score panel. The test can be performed without a grader at low cost. The apparatus can reduce the possibility of mistaken answers by the subject. In addition, the apparatus can eliminate the possibility of scoring (adding or calculating) errors caused by miscounting and the like by a grader and recording errors in the record (such as a graph). Furthermore, the scoring operation can be accurately and speedily performed. Namely, the accurate and reliable MMPI (Minnesota Multiphasic Personality Inventory) can be performed.

Furthermore, the invention is a personality testing apparatus for testing personality of a subject. Since the apparatus includes the display means 1 for displaying a MMPI (Minnesota Multiphasic Personality Inventory) question item 10 to the subject, the input means 2 for inputting an answer of the subject to the question item 10, the storage means 31 for storing test data obtained from the answer by the subject, the data processing means 3 for reading out the test data, calculating the test data according to a scale, and preparing a result table 20 obtained by aggregating the calculation result, and the output means 4 capable of outputting the result table 20 as a printed material 30, aggregation and scoring can be performed without the conventional scoring apparatus such as the scoreboard and the score panel. The test can be performed without a grader at low cost. The apparatus can reduce the possibility of mistaken answers by the subject. In addition, the apparatus can eliminate the possibility of the scoring (adding or calculating) errors caused by miscounting and recording errors in the record (such as a graph) made by a grader. Furthermore, the scoring operation can be accurately and speedily performed. Namely, the accurate and reliable MMPI (Minnesota Multiphasic Personality Inventory) can be performed. Furthermore, the result table 20 can be kept as the printed material 30.

Furthermore, because the apparatus includes the data processing means 3 for calculating the validity scale and the clinical scale from the test data obtained from the answers by the subject to the question item 10 and for preparing the result table 20 obtained by aggregating the calculation result, the validity scale and the clinical scale can be speedily aggregated and scored without using the conventional scoring apparatus such as the scoreboard and the score panel. The test can be performed without a grader at low cost. The apparatus can reduce the possibility of mistaken answers by the subject. In addition, the apparatus can eliminate the possibility of the scoring (adding or calculating) errors caused by miscounting and recording errors (for example in a graph) made by the grader. Furthermore, the scoring operation can be accurately and speedily performed. Namely, the accurate and reliable MMPI (Minnesota Multiphasic Personality Inventory) can be performed.

Furthermore, because the question item 10 corresponds to the calculation of a plurality of the scales, the number of question items 10 can be reduced. That is, the test time can be shortened and the test can be performed without burden to the subject.
Moreover, because the apparatus includes the voice output means 5 for conveying the question item 10 to the subject through voice, misreading of the subject is inhibited (misreading of Chinese characters and the like), thereby implementing an accurate test.

While many advantages of the present invention encompassed in the specification has been described above, it will be understood that the disclosure is only an illustrative example in many aspects. Without departing from the scope of the invention, various changes may be made in detail, in particular, to the shape, size, and placement of each part, the layout of the display screen on the display means, and the like.
It is to be understood that the scope of the invention is limited to the appended claims.

## Claims

1. A personality testing apparatus for testing personality of a subject, the apparatus comprising:
display means (1) for displaying a question item (10) of MMPI (Minnesota Multiphasic Personality Inventory) to the subject;
input means (2) for inputting an answer of the subject to the question item (10);
storage means (31) for storing test data obtained from the answer of the subject; and
data processing means (3) for reading out the test data, calculating the test data according to a scale, and preparing a result table (20) obtained by aggregating the calculation result.

2. A personality testing apparatus for testing personality of a subject, the apparatus comprising:
display means (1) for displaying a question item (10) of MMPI (Minnesota Multiphasic Personality Inventory) to the subject;
input means (2) for inputting an answer of the subject to the question item (10);
storage means (31) for storing test data obtained from the answer of the subject;
data processing means (3) for reading out the test data, calculating the test data according to a scale, and preparing a result table obtained by aggregating the calculation result (20); and
output means (4) capable of outputting the result table (20) as a printed material (30).

3. The personality testing apparatus according to Claim 1 or 2, wherein the data processing means (3) calculates a validity scale and a clinical scale from the test data obtained from the answer of the subject to the question item (10), and prepares the result table (20) obtained by aggregating the calculation result.

4. The personality testing apparatus according to Claim 1, 2, or 3, wherein the question item (10) corresponds to the calculation of a plurality of the scales.

5. The personality testing apparatus according to Claim 1, 2, 3, or 4 further comprising voice output means (5) conveying the question item (10) to the subject through voice.
